# EUROPEAN PATENT APPLICATION

(11) **EP 1 447 014 A1**
(43) Date of publication of application: **18.08.2004**
(21) Application number: 04003174.2
(22) Date of filing: 12.02.2004
(51) Int. Cl.: A23L 1/308, A23L 1/09, A61P 3/10, A21D 2/18, A23L 1/29, A23L 2/02

(54) **Method for reducing the glycemic index for food**

(30) Priority: 14.02.2003 EP 03003463
(71) Applicant: Wacker-Chemie GmbH, 81737 München (DE)
(72) Inventor: Schmid, Gerhard, Dr., 82131 Gauting (DE); Reuscher, Helmut, Dr., Onsted MI 49265 (US); Antlsperger, Gerard, Dr., 85598 Neubaldham (DE)
(74) Representative: Potten, Holger

(57) **Abstract**

The glycemic index of food is reduced by incorporating alpha-cyclodextrin in such food or by consuming alpha-cyclodextrin singly or in combination with other materials together with such food.

## Description

The invention relates to a method for reducing the glycemic index of food and to food with a reduced glycemic index thus produced.

Alpha-cyclodextrin is a cyclic oligosaccharide which consists of six α-(1,4)-linked anhydroglucose units. It is freely soluble in water and yields clear, colorless and tasteless solutions of low viscosity (similar to sucrose).

Only limited use has been made so far of cyclodextrins (al- pha-, beta-, gamma-cyclodextrin) for the formulation of foods (Pszczola E. (1988), Production and potential food applications of cyclodextrins. Food Techn. 42: 96-100.; Allegre M. and Deratani A. (1994), Cyclodextrin uses: from concept to industrial reality. Agro Food Industry Hi-Tech 5 (1): 9-17.; Hedges A.R., Shieh W.J. and Sikorski C.T. (1995), Use of cyclodextrins for encapsulation in the use and treatment of food products. ACS Symposium Series No. 590: 60-71). Where a use is described, it refers in most cases to beta-cyclodextrin and such use almost always is related to the property of cyclodextrins to form inclusion complexes with certain lipophilic food ingredients, such as γ-linolenic acid (abstracts of JP8187060, JP6343419, JP6153860, JP61233625, JP62011072, JP5336922), α-linolenic acid (abstract of JP7115934), omega-3 polyunsaturated fatty acids (Yoshii H., Furuta T., Kawasaki K., Hirano H., Funatsu Y., Toyomi A. and Nakayama S. (1997),. Oxidative stability of powdery tridocosahexaenoin included in cyclodextrin and its application to fish meal paste, Biosci. Biotech. Biochem. 61 (8): 1376-1378; Yoshii H., Furuta T., Ya-sunishi A., Linko Y.-Y. and Linko P. (1996), Oxidation stability of eicosapentaenoic and docosahexaenoic acid included in cyclodextrins, in: Proceedings of the Eighth International Symposium on Cyclodextrins, Szejtli J. and Szente L. (eds.), p. 579-582), conjugated linolenic acid (Park C.W., Kim S.J., Park S.J., Kim J.H., Kim J.K., Park G.B., Kim J.O. and Ha Y.L. (2002), Inclusion complex of conjugated linoleic acid (CLA) with cyclodextrins, J. Agricul. Food Chem. 50 (10): 2977-2983), rutin (abstract of JP59232054), allyl isothiocyanate (Ohta Y., Takatani K.-I. and Kawakishi S. (1995), Suppression by dextrins and polysaccharides of the decomposition of allyl isothiocyanate in aqueous solution, Nippon Nogeinagaku Kaishi (J. Agricul. Chem. Soc. Japan) 69 (9): 1175-1177; abstract of JP8245302), flavors (US20020122870, CA2111474, Kollengode A.N.R. and Hanna M.A. (1997), Cyclodextrin complexed flavors retention in extruded starches. J. Food Sci. 62 (5): 1057-1060, CA2013485), natural colors (Szente L., Mikuni K., Hashimoto H. and Szejtli J. (1998), Stabilization and solubilization of lipophilic natural colorants with cyclodextrins, J. Incl. Phenomen. Molec. Recogn. Chem. 32: 81-89, CA2112277), intense sweeteners (CA1190430, CA2006304) and unpleasant odors (US4267166; abstracts of JP2265445, JP3220117, JP6016514). In all of these applications, alpha-cyclodextrin is used as a carrier or complexant, i.e., as an auxiliary component of a composed food ingredient. In chewing gum, cyclodextrins may be used for their malodor binding properties (US4267166).

Cyclodextrins, may be contained in compositions used for the surface treatment of unprocessed or processed foodstuffs. In these compositions, cyclodextrins act as complexing agents for certain active ingredients such as ferulic acid (CA2140170) or antimicrobial agents (CA2293651, CA2295124).

The use of beta-cyclodextrin in pet food at levels of 1-15% has been disclosed for the purpose of reducing the body weight of pets (abstract of WO0021382). Unlike β-cyclodextrin, alpha-cyclodextrin administered at levels of 5 g/kg body weight for up to 13 weeks did not reduce the body weight of rats and dogs (WHO (2002), α-Cyclodextrin. 57^{th} meeting of the Joint FAO/WHO, WHO Geneva, WHO Food Additives, Series: 48: p. 111-127).

The glycemic index (GI) is a measure of the blood glucose rising property of food. It is determined by analysing the blood glucose levels in regular intervals for a 2-3 hour period after intake of the test food and a reference food which, by convention, is either white bread or glucose. The ingested amount of test food and reference food should provide the same amount of available (i.e., digestible) carbohydrate, typically 50 g. The areas under the blood glucose curves of the test food and reference food are determined. The GI of the test food is expressed as the ratio of the area under the curve of the test food and the reference food multiplied by a factor of 100, or, in other words, in percent of the GI of the reference food. The reference food, i.e., white bread or glucose, has by definition a value of 100 (FAO (1998), Carbohydrates in Nutrition, FAO Food and Nutrition Paper 66, p. 25-30).

If white bread is used as the reference food, the GI of the test food must be multiplied by a factor of 1.4 in order to obtain its GI on the glucose = 100 scale. Foods are considered to have a high, medium or low glycemic effect if their GI is, respectively, ≥70, 56-69 or ≤55 relative to that of white bread = 100 (Brand-Miller J. and Foster-Powell K. (1999); Diets with a low glycemic index: from theory to practice; Nutrition Today 34 (2): 64-72).

Since the chronic and excessive consumption of high glycemic food is associated with an increased risk of diabetes, hyperlipemia, hypertension and atherosclerosis, food with a low glycemic index is to be favored over food with a high glycemic index (Augustin L.S., Franceschi S., Jenkins D.J.A., Kendall C.W.C. and La Vecchia C. (2002); Glycemic index in chronic disease: a review. Eur. J. Clin. Nutr. 56: 1049-1071). People with a pre-diabetic condition or patients with clinically manifest diabetes are advised particularly to consume low glycemic food.

In recognition of the health benefits of low glycemic foods, different attempts have been made to lower the glycemic index of food. In most cases, it was attempted to achieve this objective by replacing food carbohydrates of high glycemic index (e.g., starch, glucose syrup, sucrose) by ingredients of lower glycemic index (e.g., high-amylose corn starch, fructose syrup, polyols). There also have been attempts to reduce the digestibility and thus the GI of starch by chemical or physical modification or by coating to make it less accessible to the digestive enzymes (US5246723; EP0749697; US5695803). Unfortunately, these starch or sugar substitutes have technological, organoleptic (sensory), or physiological properties which limit their application, be it with regard to the type(s) of food in which they can be applied without negatively affecting the taste and texture of the food, or be it with regard to the acceptable use levels in a given type of food.

Another approach for reducing the glycemic index of food relies on the addition of water-soluble, natural or synthetic dietary fibers of high viscosity (US 2002/0012733). The observation of a slower absorption of glucose from viscous solutions forms the basis of this approach. A high viscosity is only provided by dietary fibers which have a high molecular weight such as polysaccharides from different plant gums or alginates (US 2002/0172743). Oligosaccharides (such as cyclodextrins) are not expected to lower postprandial blood glucose levels by means of their viscosity which is to low for this purpose.

Unfortunately the high viscosity of the dietary fibers that have been proposed for reducing of the GI of food, such as guar gum, konjac glucomannan, etc., makes their incorporation in many types of food technologically difficult or impossible, and it also impairs the taste and mouthfeel of the foods. Furthermore, the reduction of the glycemic index that can be achieved by the use of viscous dietary fiber at technologically and organoleptically feasible concentrations, is modest.

With the currently available food ingredients it is, therefore, still not possible to significantly lower the glycemic index of many types of food, in particular of starch-based foods, and liquid or semiliquid foods, without compromising their texture, stability, viscosity, taste, appearance and/or nutritional value.

The present invention relates to a method to modify food having a glycemic index characterised in that the glycemic index of the food is reduced by combining the food with alpha-cyclodextrin in an effective amount.

For the purpose of the present invention the term "glycemic index" GI) of a food is defined by the incremental area under the blood glucose response curve of that food expressed in percent of the response to the reference food expressed as a percentage of the response to the reference food (with the same amount of available carbohydrate) which, by convention, is fresh white bread or glucose. As there exists a close association between the glycemic and insulinemic response to food, in the present application the term "glycemic index" encompasses the term "insulinemic index". Correspondingly, the terms "low-", "medium-" and "high-glycemic" mean, interchangeably, "low-", "medium-" and "high-insulinemic".

For the purpose of the present invention the terms "low-glycemic", "medium-glycemic" and "high-glycemic" food are used to designate food with a GI of ≤55, 56-69 and ≥70, respectively (e.g. white bread = 100).

For the purpose of the present invention the term "food" means any substance or product, whether processed, partially processed or unprocessed, intended to be, or reasonably expected to be consumed by humans or animals for its nutritive properties or pleasure. It includes foods for special dietary purposes (such as foods positioned for consumption by diabetics) and foods for medical purposes (such as formula diets for tube feeding). Preferably the term "food" means starch-based food, especially preferred starch-based food having a GI of ≥ 55.

Since the chronic ingestion of high-glycemic food bears health risks not only for humans but also for animals, notably those who live till the end of their natural life span (e.g., pet animals), this invention pertains also to food destined for consumption by animals, in particular companion animals. Preferably this food has a fat content of less than 5% by weight.

Preferably food means such food which is taken up in a portion size of ≥ 15g or ≥ 15ml.

Especially preferred the term food means food destined for consumption by healthy humans or humans suffering from diabetes, prediabetic conditions, metabolic syndrom ("Syndrom X"), overweight, hypertension, hyperlipidemia or other physiological or medical disorders.

Unless indicated otherwise, all "use levels" referring to alpha-cyclodextrin, are expressed in percentages, and are calculated by weight.

The present invention provides a method for lowering the GI of foods by use of alpha-cyclodextrin at levels sufficient to achieve this intended effect. This method enables consumers to control their postprandial blood glucose and thereby insulin levels, without restricting their choice of food and without compromise on the palatability, appearance and quantity of the food consumed. Since alpha-cyclodextrin is tasteless, odourless, of low viscosity and well tolerated even at high intakes, its incorporation in foods and/or its consumption with food does not pose technological problems and does not reduce food acceptance. In all uses of alpha-cyclodextrin cited as state of the art, except chewing gum, alpha-cyclodextrin is first reacted with the flavors, colors, nutrients etc. with which it forms an inclusion complex or aggregate. Since the described flavors, colors, nutrients, etc. are incorporated in foods only in small amounts and since the ratio of complexed substance to alpha-cyclodextrin ranges typically from about 1:1 to 1:5 (w/w), the concentration of alpha-cyclodextrin from its use as a complexant, such uses in the final food does not exceed 1%, except in chewing gum in which higher concentrations may be found. Under these conditions of use, the consumption of alpha-cyclodextrin does not reach the levels of intake which are required for producing a glycemia-lowering effect, either because the alpha-cyclodextrin concentration in the food is to low and/or because the food is consumed in only small amounts (chewing gum).

Pursuant to the present invention, alpha-cyclodextrin is consumed in or together with individual foods or whole meals. This is achieved by direct incorporation of uncomplexed alpha-cyclodextrin in a food during its manufacturing process or during its preparation e.g. at home or by mass-caterers in an amount of 0,1-30% (w/w). Therefore in a preferred embodiment of the present invention the food is combined with the alpha-cyclodextrin by an incorporation of the alpha-cyclodextrin in the food during its manufacturing process.

Preferably the food is turned from a high-glycemic food to a medium or low-glycemic food, or from a medium-glycemic food to a low-glycemic food by the incorporation of alpha-cyclodextrin. In this embodiment of the invention preferably more than 40% of the calories of the food are provided by carbohydrates, especially preferred more than 50% .

Alternatively, an alpha-cyclodextrin containing food which in itself preferably is low- or non-glycemic, (e.g. an alpha-cyclodextrin containing diet soft drink) may be consumed prior to, together with, or after any food and thereby reduce the glycemic impact of this food. Sufficient levels of alpha-cyclodextrin will be ingested with foods that have a serving size of ≥ 15 g for solid foodstuffs or ≥ 15 ml for liquid foodstuffs. The same effect will be achieved by consumption of alpha-cyclodextrin in the form of a supplement (e.g., in the form of a capsule, tablet, powder, liquid or other pre-dosed form) prior to or together with food.

So, it is possible to lower the glycemic index of a food also by combining the food with the alpha-cyclodextrin before, together with or after the uptake of such food. This can e.g. be done by ingesting an alpha-cyclodextrin or an alpha-cyclodextrin containing beverage or other formulation prior to, during or after consumption of such food.

If alpha-cyclodextrin is taken up prior to uptake of the food, it is preferably taken up about 0.01 to 0.5 hours prior.

If alpha-cyclodextrin is taken up after uptake of the food, it is preferably taken up about 0.01 to 0.5 hours after consumption of the food.

Preferably the alpha-cyclodextrin is taken up prior to or during consumption of the food. Especially preferred the alpha-cyclodextrin is taken up during consumption of the food.

In this embodiment of the invention the alpha-cyclodextrin is preferably taken up as uncomplexed alpha-cyclodextrin in a solid formulation (e.g. capsules, tablets, powder) with or without the use of formulation aids. Repective formulation aids are known to someone skilled in the art Alternatively the alpha-cyclodextrin can also be taken up as uncomplexed alpha-cyclodextrin in a liquid formulation.

Evidently, the biggest benefit is drawn from the present invention if alpha-cyclodextrin is consumed in or together with food of high or medium GI thereby reducing its GI to a medium or low level, respectively. The incorporation of alpha-cyclodextrin into food is, therefore, particular recommended for foods with a high starch content. Alpha-cyclodextrin is therefore preferably incorporated into breads and rolls, muffins, brownies, cakes, crackers (sweet and non-sweet), cereal bars, quick breads, dough and baking mixes, as well as sweet and savory snacks, ready-to-eat breakfast cereals, instant rice, baked, mashed and boiled potatoes, french fries, pasta and noodles. Because of its low-viscosity, alpha-cyclodextrin can be used for its GI-lowering effect also in liquid formula diets, such as meal-replacements and enteral formula diets (for tube feeding).

According to the present invention, a GI-lowering effect is also achieved by consuming a combination of a low-glycemic, alpha-cyclodextrin containing food together with a regular, i.e., not modified, medium- or high-glycemic food. Accordingly, it is also preferred to incorporate alpha-cyclodextrin in low-glycemic foods include all types of beverages (e.g., diet soft drinks, fruit juices, vegetable juices, dry beverage mixes), soy and non-soy (imitation) milk, milk-based drinks as well as canned or dry soups, bread-spreads and yoghurt.

If alpha-cyclodextrin is used in uncomplexed form, the alpha cyclodextrin is preferably incorporated in concentrations of 0.1 - 30% (w/w), especially preferred in concentrations 1-15% (w/w), especially preferred in concentrations of 2-10% (w/w) .

Since alpha-cyclodextrin is chemically inert and does not materially affect the viscosity and texture of foods, it is possible to combine alpha-cyclodextrin with other ingredients which may support its GI-lowering effect. Depending upon the food matrix, it is, for example, possible to include additional amounts of low-digestible carbohydrates (e.g., fructo-oligosaccharides, inulin, dextrins, polydextrose, betaglucans, resistant starch, D-tagatose, polyols), chromium salts, medium-chain triglycerides and diacylglycerol.

If it is used in complexed form, the alpha cyclodextrin is preferably incorporated in concentrations of 3 - 30% (w/w), especially preferred in a concentrations of 5 - 15% (w/w).

If the alpha cyclodextrin is in complexed form, the cyclodextrin is preferably complexed with digestible or partially indigestible, natural or synthetic oils or fats.

In order to best achieve one objective of the method according to this invention, namely to reduce the postprandial blood glucose response and thereby improving the control of blood glucose levels and potentially reducing and/or delaying the need for blood glucose lowering medication, it may be necessary to communicate these benefits to consumers either directly through information on the food label or accompanying leaflets or indirectly through advertisements, recommendations by health professionals or other means of communications. Even if such communication does not refer explicitly to the lower glycemic index of a food containing alpha-cyclodextrin or the glycemia lowering effect of a supplement containing alpha-cyclodextrin, but instead uses other terms, pictures, symbols or the like conveying the same message, such products are contemplated to fall within the scope of this invention.

The lowering of the glycemic index of food entails a lowering of the insulin secretion following the ingestion of such food, thereby exerting benefits in terms of the delaying of aging processes and the prevention of hyperlipidemia, hypertension, obesity, artherosclerosis, diabetes, and the manifold pathological consequences of these conditions. The present invention, therefore, also pertains to food which owing to its alpha-cyclodextrin content as described herein is positioned for delaying of aging and the prevention or risk reduction of one or several of the mentioned ailments.

The food according to the present invention preferably comprises alpha cyclodextrin in a concentration of 0,1-30% (w/w), preferred in a concentration of 1 - 15% (w/w), especially preferred in a concentration of 2-10% (w/w).

Further examples of food which benefits from the method according to the present invention are listed below:
- Bakery products, e.g. breads and rolls, brownies, cakes (light weight), crackers (used as snacks), grain-based bars (e.g. breakfast bars, granola bars, rice cereal bars), coffee cakes, crumb cakes, sweet rolls, muffins, refrigerated dough, dry baking mixtures;
- Beverages, e.g. dry mixes for beverages, dry powder fruit flavored beverage mixes, carbonated and noncarbonated sugarfree (diet) soft drinks,;
- Cereal and other grain products, e.g. breakfast cereals, ready to eat types, instant rice, pastas
- Dairy products, e.g. yogurt, including yogurt beverages and frozen yogurt, dry mixes for pudding, dry mixes for milk-based beverages;
- Fats and oils, e.g. margarine, bread spreads;
- Fruit and fruit juices, e.g. juices, nectar, fruit drinks, including fruit milk drinks;
- Sauces, e.g. spaghetti sauce, ketchup;
- Snacks, all types, e.g. savory snacks, sweet and non sweet crackers, potato chips;
- Soups, e.g. canned soups, dry mixes for soups, e.g. dry bouillon, instant soups etc;
- Vegetables, e.g. vegetable juice, vegetable sauces or purees, e.g. tomato sauce, tomato puree, soy milk.

Since alpha-cyclodextrin at the use levels required to achieve the intended GI-lowering effect has a low viscosity, it does not compromise the texture, stability, taste and appearance of the food in which it is incorporated. Due to its low viscosity, alpha-cyclodextrin can also be incorporated in liquid foods including beverages, lipid meal replacements, and formula diets for enteral application (tube feeding).

Another advantage of alpha-cyclodextrin is that it is well tolerated even at high levels of intake and does not lead to the intestinal symptoms (flatulence, bloating, diarrhea) which are typically associated with the ingestion of high amounts of low digestible and thus low-glycemic carbohydrates such as polyols, fructo-oligosaccharides, inulin, or soluble dietary fibers of high viscosity.

According to this invention, alpha-cyclodextrin is added to food or consumed together with food, without necessarily substituting for other components, notably the high-glycemic components of such food. The beneficial effects of alpha-cyclodextrin used according to this invention are, therefore, brought about by a reduction of the glycemic index of the thus prepared foods but not necessarily by a reduction of its nutritional and/or energetic value.

It is therefore also part of the present invention to use alpha-cyclodextrin to reduce the glycemic index of food. It is a further part of the present invention to use alpha-cyclodextrin as supplement in combination with the intake of food to reduce the glycemic index of such food. At present, alpha-cyclodextrin is used in foods only for technological reasons, namely as a complexant for a number of volatile or chemically susceptible food ingredients. The incorporation of complexes of these ingredients with alpha-cyclodextrin results in very low concentrations of complexed alpha-cyclodextrins in foods. At this low level of typically <1%, alpha-cyclodextrin cannot be expected to affect the GI of such foods.

Alpha-cyclodextrin may be incorporated in food by a variety of methods known to those skilled in the art. These methods include the mixing of alpha-cyclodextrin in the dough of bread and other baked goods, the mixing of alpha-dextrin in fillings or glazings of baked good, the incorporation of alpha-cyclodextrin in snack wavers prior to extrusion, the incorporation of alpha-cyclodextrin in breakfast cereals or its application, with or without the addition of sugar(s), on the surface of the cereals; the mixing of alpha-cyclodextrin into the dough of pasta and noodles; the dissolution of alpha-cyclodextrin in the water in which rice is boiled; the admixture of alpha-cyclodextrin to dry instant soup, instant rice instant past and muesli mixtures, and the dissolution of alpha-cyclodextrin in canned soup and beverages. In foods which contain a significant amount of lipids, such as digestible or partially indigestible oils and fats, it may be advantageous to first form a complex of the oil and/or fat with alpha-cyclodextrin by kneading or vigorous mixing, or other means and to then incorporate the complex in the food.

While the incorporation of alpha-cyclodextrin in food is the preferred manner of implementing the method revealed in the present invention, it may sometimes be more practical to consume alpha-cyclodextrin in the form of a tablet, capsule, powder, syrup, stabilized dispersion or similar dry or liquid formulation designed to be consumed in measured quantities. Such embodiments of alpha-cyclodextrin may be positioned as foods, food supplements or medicinal preparations. They may contain, in addition to alpha-cyclodextrin, other useful ingredients such as vitamins and minerals, as well as food additives or excipients which are required for ensuring the stability, free-flowing properties (in the case of powders), appearance and taste of the formulation. According to the present invention, such formulations are consumed preferably prior to or together with food or a meal. Powders or liquid formulations may also be mixed into the regular foods, be it during preparation or immediately prior to consumption.

Pet animals are often suffering from the same diet-related ailments as their human proprietors. Diabetes and excessive fat deposition are frequently diagnosed in aged dogs, cats, rabbits and other pets. Therefore, the administration of food with a reduced glycemic index is beneficial also for pets.

According to the present invention, alpha-cyclodextrin may be incorporated in food destined for consumption by pet animals as described for human foods. Food with a high starch content, such as "snack"-type products, processed cereals and flour-based cookies, are preferred applications. However, alpha-cyclodextrin may also be admixed to canned food at levels which are sufficient to achieve the intended effect.

The following examples are provided to illustrate the invention and are not intended to limit its scope in any way:

### Example 1

12 healthy, overnight fasted, male volunteers received on separate days, in random order, a test "breakfast" consisting of (A) 100 g fresh white bread (providing 50 g starch) together with a beverage (250 ml tap water), or (B) 100 g fresh white bread together with an alpha-cyclodextrin containing beverage (10 g alpha-cyclodextrin dissolved in 250 ml tap water). Capillary and venous blood was collected before start of the breakfast and thereafter in regular intervals for a period of 3 hours. Plasma glucose was determined in the capillary blood and plasma insulin in the venous blood samples.

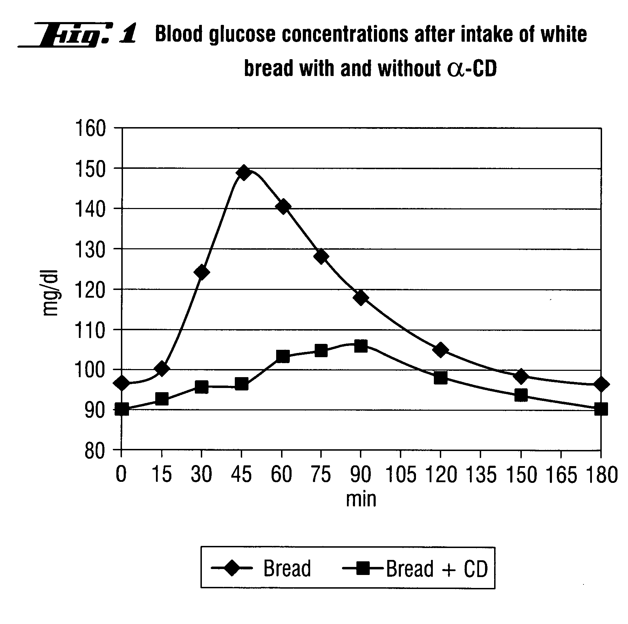

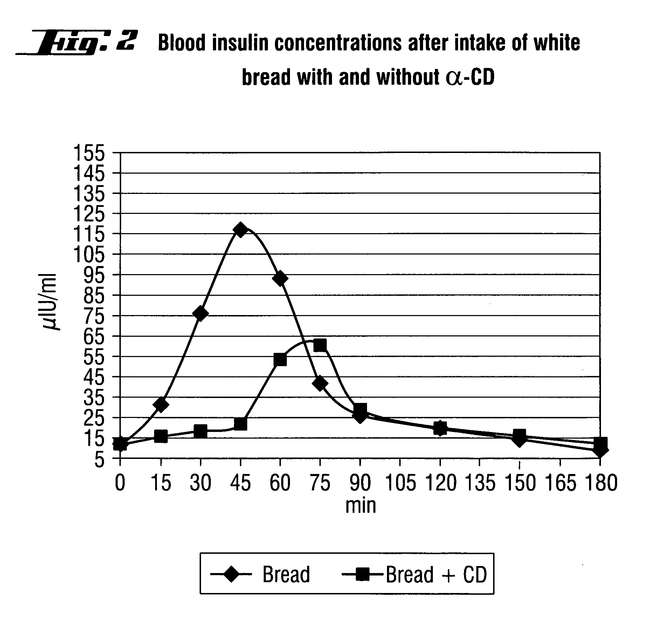

Breakfast (A) led to the expected rise in blood glucose and insulin concentrations. The postprandial rise of plasma glucose and insulin was significantly smaller after breakfast (B) than (A) (Figs. 1, 2). Under the influence of alpha-cyclodextrin, the glycemic and insulinemic index of white bread was reduced by 57 and 55%, respectively. The time profile of plasma glucose and insulin after breakfast (B) demonstrates that, in an initial phase, there is almost no rise in blood glucose and insulin levels. This delay in the absorption of glucose reflects a prolonged presence of carbohydrate in the stomach and/or intestinal lumen. This effect together with a lower rise of blood glucose concentration and the absence of a rebound effect (a fall of blood glucose levels below baseline levels) may result in a longer lasting satiety which would be an additional benefit of the present invention, particularly for overweight consumers and pre-diabetic or diabetic subjects. The intake of breakfast (B) was tolerated very well and was not associated with flatulence or any other gastrointestinal symptoms.

### Example 2

Low-glycemic bread rolls are prepared using the following ingredients in the indicated amounts. The composition contains about 7% alpha-cyclodextrin. The product has a glycemic index of <55.

| Ingredient | amount |
|---|---|
| Flour | 550 g |
| Alpha-cyclodextrin | 50 g |
| Milk powder | 15 g |
| Salt | 11 g |
| Sugar | 15 g |
| Fat | 15 g |
| Yeast | 26 g |
| Water | approx. 45 g |

The rolls are prepared by mixing the flour, fat, yeast (suspended in water and sugar and kneading it to a smooth and elastic dough. The dough is kept at a warm place until its volume has approximately doubled in size. The dough is divided into 60 g pieces which are formed as desired. The pieces are placed on a tray and left in a warm place to prove. Baking in an oven at 200-250 °C for 12-15 minutes yields the final product ready for consumption.

### Example 3

A fat reduced bread spread which, if consumed with bread, will reduce the glycemic index of the latter, is prepared as follows:

| Ingredient | amount |
|---|---|
| Sunflower oil | 20% |
| Rapeseed oil | 20% |
| Alpha-cyclodextrin | 10% |
| Butter | 10% |
| Flavor | 0.1% |
| Salt | 0.1% |
| Potassium sorbate | 0.1% |
| Whey | q.s. ad 100% |

The oils and fat-soluble flavors are mixed. Alpha-cyclodextrin is added under vigorous mixing whereby inclusion complexes are formed. Salt and potassium sorbate are dissolved in whey. The solution is pasturized and mixed with the oil/alphacyclodextrin mixture and butter in a cooled kneading machine. The product is an oil-in-water emulsion with a good spreadibility.

### Example 4

A liquid formula diet with a reduced glycemic index designed for consumption by type-1 and type-2 diabetics and persons with stress-induced hyperglycemia or other conditions of abnormal glucose tolerance is prepared using the following ingredients:

| Ingredient | amount |
|---|---|
| Caseinates | 5.0% |
| Maltodextrin | 5.9% |
| Fructose | 5.5% |
| Soy polysaccharide | 0.5% |
| Safflower oil | 1.8% |
| Canola oil | 1.8% |
| Alpha-cyclodextrin | 1.3% |
| Vitamin and mineral premix Nutrients (L-carnitine, | 0.1% |
| inositol, choline, taurine) | 0.1% |
| Lecithin | 0.1% |
| Water | q.s. ad 100% |

### Example 5

A low-glycemic beverage which, when consumed prior to or together with starch containing food, will reduce the glycemic index of the latter, is prepared as follows:

| Ingredient | amount |
|---|---|
| Fructose | 6% |
| Alpha-cyclodextrin | 3% |
| Orange juice concentrate | 1% |
| Citric acid | 0.2% |
| Flavors | 0.1% |
| Ascorbic acid | 0.05% |
| Water | q.s. ad 100% |

### Example 6

Cooked rice with a low glycemic index is obtained by adding 1 kg rice and 80 g alpha-cyclodextrin to 2.8 litre boiling water followed by cooking at about 90°C until the water is absorbed.

### Example 7

50 ml of milk are added to 60 g of breakfast cereal with a total carbohydrate content of 44g. To reduce the glycemic index of this breakfast two sachets filled with 2.5 g of alpha cyclodextrin each are opened and the contents sprinkled over the cereal.

## Claims

1. A method to modify food having a glycemic index **characterised in that** the glycemic index of the food is reduced by combining the food with alpha-cyclodextrin in an effective amount.

2. The method according to claim 1 **characterised in that** the food is combined with the alpha-cyclodextrin by an incorporation of the alpha-cyclodextrin in the food during its manufacturing process.

3. The method according to claim 1 **characterised in that** the food is combined with the alpha-cyclodextrin before, together with or after an uptake of such food.

4. The method according to one of claims 1 to 3 **characterised in that** the alpha-cyclodextrin is uncomplexed and used in concentration of 0.1 - 30% (w/w).

5. The method according to one of claims 1 to 3 **characterised in that** the alpha-cyclodextrin is complexed and used in concentration of 3 - 30% (w/w).

6. The method according to claim 5 wherein alpha-cyclodextrin is complexed with digestible or partially indigestible, natural or synthetic oils or fats.

7. The method according to one of claims 1 to 6 wherein the food is destined for consumption by healthy humans or humans suffering from diabetes, prediabetic conditions, metabolic syndrom ("Syndrom X"), overweight, hypertension, hyperlipidemia or other physiological or medical disorders.

8. The method according to one of claims 1 to 6 **characterised in that** the food is destined for consumption by pet animals.

9. The method according to claim 8 wherein the food has a fat content of less than 5% by weight.

10. A food which owing to its alpha-cyclodextrin content is positioned for the prevention or risk reduction of an ailment selected from the group hyperlipidemia, hypertension, obesity, artherosclerosis, diabetes, and the manifold pathological consequences of these conditions.

11. A food made by the method according to claim 2 **characterised in that** it is selected from bakery products, beverages, cereal and other grain products, dairy products, fats and oils, vegetable and fruit juices, sauces, snacks, soups, liquid meal replacements, and foods for medical purposes.

12. The food according to claim 10 or 11 **characterised in that** it comprises alpha cyclodextrin in a concentration of 0,1-30% (w/w), preferred in a concentration of 1 - 15% (w/w), especially preferred in a concentration of 2-10% (w/w).

13. A process for the production of food according to one of claims 10 to 12 **characterised in that** alpha cyclodextrin is incorporated into the food in a concentration of 0,1-30% (w/w) during its manufacturing process or during its preparation.

14. Use of alpha-cyclodextrin to reduce the glycemic index of food.

15. Use of alpha-cyclodextrin as supplement in combination with the intake of food to reduce the glycemic index of such food.
